# EUROPÄISCHE PATENTSCHRIFT

(11) **EP 3 188 766 B1**
(45) Veröffentlichungstag und Bekanntmachung des Hinweises auf die Patenterteilung: **17.04.2019**
(21) Anmeldenummer: 15763841.2
(22) Anmeldetag: 26.08.2015
(51) Int. Cl.: A61L 27/50

(54) **FORMKÖRPER ZUR NACHBILDUNG EINER STRUKTUR EINES BIOLOGISCHEN GEWEBES UND VERFAHREN ZU DESSEN HERSTELLUNG**
MOULDING FOR REPLICATING A STRUCTURE OF A BIOLOGICAL TISSUE AND METHOD FOR PRODUCING THE SAME
CORPS DE MOULE POUR SIMULER UNE STRUCTURE D'UN TISSU BIOLOGIQUE ET SON PROCÉDÉ DE FABRICATION

(30) Priorität: 03.09.2014 DE 102014112660
(43) Veröffentlichungstag der Anmeldung: 12.07.2017
(73) Patentinhaber: Technische Universität Ilmenau, 98693 Ilmenau (DE)
(72) Erfinder: SCHOBER, Andreas, 99096 Erfurt (DE); HAMPL, Jörg, 99090 Erfurt (DE); HÄFNER, Sebastian, 01217 Dresden (DE); TOBOLA, Justyna, 98693 Ilmenau (DE); WEISE, Frank, 98693 Ilmenau (DE); SINGH, Sukhdeep, 98693 Ilmenau (DE); SCHLINGLOFF, Gregor, 98693 Ilmenau (DE)
(74) Vertreter: Engel, Christoph Klaus
(86) Internationale Anmeldenummer: PCT/EP2015/069522
(87) Internationale Veröffentlichungsnummer: WO 2016/034471

(56) Entgegenhaltungen:
- EP-A2- 2 650 256
- DE-A1-102012 101 240
- US-A1- 2010 098 742

## Beschreibung

Die vorliegende Erfindung betrifft zunächst ein Verfahren zur Herstellung eines Formkörpers zur Nachbildung einer Struktur eines biologischen Gewebes. Der Formkörper dient insbesondere der Ansiedlung von Zellen und weist entsprechende Gefäße auf, sodass beispielsweise das Gewebe einer Leber oder eine Blut-Hirn-Schranke nachgebildet werden kann. Im Weiteren betrifft die Erfindung einen Formkörper zur Nachbildung einer Struktur eines biologischen Gewebes.

Biologische Systeme, insbesondere auch Organismen wie Tiere und Menschen besitzen unterschiedliche Organe, die aus verschiedenen Zelltypen bestehen und trotz einer gewissen Varianz unterschiedlicher Individuen eine wohldefinierte Morphologie besitzen. Ein wesentliches Kernelement stellt dabei die Vaskularisierung, d. h. die Versorgung des Gewebes mithilfe der Blutkapillaren, dar. Die unterschiedliche Funktionalität des einzelnen Organs zeigt sich dabei auch in der unterschiedlichen Struktur bzw. in den unterschiedlichen Eigenschaften der Blutkapillaren. In der Leber ist das Endothel der Blutkapillaren so aufgebaut, dass eine gewisse Durchlässigkeit im Disse-Raum realisiert wird, während im Gehirn die Blut-Hirn-Schranke so ausgeführt ist, dass ein direkter Übergang von Stoffen aus dem Blut in das Gewebe nicht möglich ist, da Endothelzellen und Perizyten eine undurchlässige Barriere bilden. Ein Beispiel ist das Grundgewebe des Knochenmarks, welches von zahlreichen dünnwandigen Blutgefäßen durchzogen ist, die als Knochenmarksinus bezeichnet werden. Die Wand der Sinusoide wird von einem zarten, unregelmäßig durchbrochenen Endothel gebildet, welches keine Basallamina besitzt. Die Kapillardichte im Gehirn, vor allem in der grauen Substanz der Großhirnrinde, ist sehr hoch. Die mittlere Entfernung zwischen einzelnen Kapillaren beträgt nur 40 µm bei einem Durchmesser von 3 µm bis 7 µm. Lebersinusoide sind Kapillaren mit einer Länge von ca. 0,5 mm und einen Durchmesser von 9 µm bis 12 µm.

Die WO 2012/045687 A1 zeigt eine Struktur zur Nachbildung eines kleinen Blutgefäßes in Form eines Sinusoids, wie es beispielsweise in einer Leber vorkommt. Zwischen mehreren übereinander angeordneten Schichten aus einem porösen Material ist jeweils ein Zwischenraum ausgebildet. Die Schichten weisen eine Kokultur der bei dem nachzubildenden Sinusoid vorhandenen Zellspezies auf. Weiterhin sind in den Schichten Kanäle ausgebildet, welche die Zwischenräume verbinden und zur Weiterleitung eines Fluids, wie Blut ausgebildet sind.

Die WO 02/053193 A2 lehrt eine mehrschichtige Struktur mit einer ersten Schicht bestehend aus einem zur Ansiedlung von tierischen Zellen geeigneten Material. Die erste Schicht weist ein Muster von Mikrokanälen auf, wobei die Kanäle zur Ansiedlung von tierischen Zellen und zur Zirkulation von Fluid durch die Schicht geeignet sein sollen. Weiterhin umfasst die Struktur mindestens eine zweite Schicht aus einem zur Ansiedlung von tierischen Zellen geeigneten Material. Die erste und zweite Schicht sind miteinander verbunden, wodurch Kanäle erzeugt werden. Die erste Schicht kann zur Ansiedlung von Endothelzellen und die zweite Schicht zur Ansiedlung von Epithelzellen, wie Hepatozyten dienen. Die Schichten können übereinander gestapelt sein und sind über Durchgangslöcher miteinander verbunden.

Aus der WO 2004/026115 A2 ist ein Verfahren zur Herstellung einer Struktur zur Nachbildung eines Sinusoids bekannt, welche mehrere gestapelte zweidimensionale Schichten besitzt, auf welchen beispielsweise Endothelzellen und Hepatocyten angeordnet sind. Die Schichten können Durchgangslöcher aufweisen.

Die US 2010/0098742 A1 zeigt ein synthetisches Gewebe, welches beispielsweise ein Blutgefäßsystem oder ein Lymphsystem bildet. Gemäß einer in den Fig. 8A bis 8C der US 2010/0098742 A1 gezeigten Ausführungsform werden zwei Hohlform- und/oder Polymergewebeteile zusammengefügt, um einen Einschluss zu bilden. Gemäß einer weiteren Ausführungsform wird vorgeschlagen, einen langen Streifen eines geformten Polymers zu falten, um eine Dreidimensionalität zu erzielen. Der Streifen des geformten Polymers umfasst ein Blutgefäßsystem und weist Durchgangsöffnungen auf. Beim Falten werden die Durchgangsöffnungen übereinander angeordnet, sodass ein Gefäß in die z-Richtung entsteht.

Die EP 2 650 256 A2 zeigt ein Verfahren zur Herstellung eines mikrostrukturierten Formkörpers, welcher zur Kultivierung von biologischen Zellen vorgesehen ist. Gemäß diesem Verfahren werden eine plastisch verformbare, poröse erste Folie, eine verformbare zweite Folie und eine verformbare Opferfolie bereitgestellt, die zu einem Folienstapel gestapelt werden. Anschließend wird die Opferfolie mit einem Druck beaufschlagt, um den Folienstapel in eine Form zu pressen. Die Form weist Ausnehmungen auf, wodurch in den Folien jeweils verformte Bereiche in Form von Kavitäten ausgebildet werden. Es erfolgt ein Ätzen der verformten Bereiche der zweiten Folie, wodurch Abschnitte der zweiten Folie chemisch aufgelöst werden, in denen die Poren wieder geöffnet werden.

Die DE 10 2012 101 240 A1 zeigt ein Verfahren zur Herstellung einer Struktur für die Ansiedlung biologischer Zellen, bei welchem ein Träger aus einem Polymer bereitgestellt wird, der mit einer versprühbaren Substanz unter Nutzung einer Maske chemisch modifiziert wird.

Die Aufgabe der vorliegenden Erfindung besteht darin, die Bildung von Gefäßen bei der Herstellung von Strukturen, welche zur Nachbildung eines biologischen Gewebes dienen, aufwandsärmer und genauer ausführen zu können.

Die genannte Aufgabe wird gelöst durch ein Verfahren gemäß dem beigefügten Anspruch 1 sowie durch einen Formkörper gemäß dem beigefügten nebengeordneten Anspruch 9.

Das erfindungsgemäße Verfahren dient zur Herstellung eines Formkörpers zur Nachbildung einer Struktur eines biologischen Gewebes. Der Formkörper bildet eine räumliche Struktur des biologischen Gewebes nach und dient insbesondere zur Aufnahme von biologischen Zellen und Flüssigkeiten. Die biologischen Zellen werden in dem Formkörper angesiedelt und bilden bevorzugt eine Komponente des Formköpers. Der Formkörper erlaubt einen Stoffaustausch, insbesondere einen Flüssigkeitsaustausch zwischen den Zellen und einem Äußeren des Formköpers. Das nachzubildende biologische Gewebe ist bevorzugt durch eine Leber bzw. Leberstrukturen, durch eine Blut-Hirn-Schranke, durch eine Knochenmarkstruktur oder durch einen Muskel bzw. eine muskuläre Struktur gebildet. Es können aber auch andere Arten von biologischem Gewebe nachgebildet werden, wie z. B. Nierengewebe oder Milzgewebe. Das biologische Gewebe kann menschlich, tierisch oder pflanzlich sein.

In einem Schritt des erfindungsgemäßen Verfahrens wird eine plastisch verformbare Folie bereitgestellt, die bevorzugt porös ist. Die Folie besteht bevorzugt aus einem biokompatiblen Material, besonders bevorzugt aus einem biokompatiblen Kunststoff.

In einem weiteren Schritt wird die Folie mit einem Druck beaufschlagt, um diese in eine Form zu pressen. Die Form weist Ausformungen für grabenartige Vertiefungen, für Mulden und für Kerben auf. Somit entstehen beim Pressen der Folie in die Form grabenartige Vertiefungen, Mulden und Kerben in der Folie. Die grabenartigen Vertiefungen werden in der Erstreckungsebene der Folie ausgeformt, sodass sich die grabenartigen Vertiefungen entlang der Erstreckungsebene der Folie erstrecken. Die Mulden grenzen jeweils zumindest an eine der grabenartigen Vertiefungen an. Die Mulden weisen jeweils einen Boden auf, an welchem sie geöffnet werden, sodass jeweils eine Öffnung entsteht. Dabei bleiben zumindest seitliche Abschnitte der Muldenform erhalten, sodass es sich um nach oben und nach unten offene Mulden handelt. Die Kerben bilden mindestens ein Filmscharnier in der Folie aus, an welchem die Folie gefaltet werden kann.

In einem weiteren Schritt erfolgt ein Falten der ausgeformten Folie zu einem mindestens zwei Schichten der Folie umfassenden Stapel. Dabei bildet das Filmscharnier die Faltkante zum Falten. Es erfolgt somit mindestens ein Faltvorgang, bei welchem die Folie am Filmscharnier um 180° geschwenkt wird, sodass zwei Abschnitte der Folie zum Aufliegen kommen und die Schichten des Stapels bilden. Die Schichten weisen bevorzugt gleiche Längen und Breiten auf und sind deckungsgleich übereinander im Stapel angeordnet. Bevorzugt erfolgen mehrere Faltvorgänge, wofür in der Folie eine entsprechende Anzahl der Filmscharniere ausgebildet wird. Dabei erfolgt das Falten bevorzugt Leporello-artig, d. h. im "Zick-Zack", abwechselnd einmal nach vorn und einmal nach hinten. Es können aber auch andere Abläufe des Faltens gewählt werden, beispielsweise durch die Bildung von Teilstapeln, die wiederum zueinander gefaltet werden, wobei die einzelnen Teilstapel in eine andere Richtung gefaltet werden als die Teilstapel zueinander. Durch das Falten werden die in einer der Schichten befindlichen grabenartigen Vertiefungen entlang ihrer Erstreckungsrichtung durch die jeweils benachbarte der Schichten des Stapels geschlossen, sodass sie jeweils eine Kapillare ausbilden. Die Kapillaren erstrecken sich in gleicher Weise in der Erstreckungsebene der Folie wie die grabenartigen Vertiefungen. Die Kapillaren bilden bevorzugt Blutkapillaren oder Leitkanäle für das Wachstum von Neuriten aus. Durch das Falten werden mindestens zwei der geöffneten Mulden übereinander angeordnet, sodass sie einen senkrecht zur Erstreckungsebene der Folie angeordneten Kanal ausbilden. Da die Mulden jeweils mit zumindest einer der grabenartigen Vertiefungen verbunden sind, sind die durch die Mulden gebildeten Kanäle auch jeweils mit zumindest einer der Kapillaren verbunden, sodass Flüssigkeiten von den Kanälen in die Kapillaren und umgekehrt fließen können. Der gefaltete Stapel bildet den herzustellenden Formkörper. Der Formkörper wird bevorzugt in ein Bioreaktorsystem eingebracht, wofür u. a. Verbindungen zu den Kanälen zu schaffen sind. Bevorzugt werden mehrere Formkörper aufeinander gestapelt und ausgerichtet, um ein komplexes System zur Nachbildung biologischer Strukturen zu bilden.

Ein besonderer Vorteil des erfindungsgemäßen Verfahrens besteht darin, dass durch die Filmscharniere ein aufwandsarmes exaktes Übereinanderanordnen der Folienabschnitte ermöglicht ist, sodass die auszubildenden Gefäße, d. h. die auszubildenden Kapillaren und Kanäle vorgabegemäß ausgebildet werden. Die Filmscharniere werden mit der gleichen Genauigkeit wie die grabenartigen Vertiefungen und die Mulden ausgeformt, sodass ein passgenaues Übereinanderanordnen gewährleistet ist. Es ist keine aufwändige Positionierung erforderlich. Insbesondere ermöglicht es das erfindungsgemäße Verfahren, dass gleichzeitig die Kapillaren und die dazu senkrecht angeordneten Kanäle ausgebildet werden, wie sie zur Nachbildung vieler biologischer Strukturen notwendig sind.

Bei bevorzugten Ausführungsformen des erfindungsgemäßen Verfahrens wird die Folie vor dem Pressen erwärmt. Insofern die Folie beispielsweise aus Polycarbonat oder aus einem vergleichbaren Material besteht, wird sie bevorzugt auf eine Temperatur zwischen 140 °C und 180 °C erwärmt, sodass sie beim Pressen diese Temperatur aufweist. Insofern die Folie beispielsweise aus einem bioabbaubaren Polymer besteht, wird sie bevorzugt auf eine Temperatur zwischen 35 °C und 80 °C erwärmt. Das Erwärmen erfolgt bevorzugt über die Form, d. h. die Folie wird indirekt über die Form erwärmt, bevor sie mit dem Druck beaufschlagt wird.

Die Form kann aus einem harten oder auch bedingt flexiblen Material bestehen. Sie besteht bevorzugt aus Silizium oder aus Polydimethylsiloxan (PDMS). Die Form kann als Positivform oder als Negativform ausgebildet sein, d. h. die Ausformungen für die grabenartigen Vertiefungen, für die Mulden und für die Kerben können erhaben oder versenkt sein.

Bei besonders bevorzugten Ausführungsformen des erfindungsgemäßen Verfahrens wird Polycarbonat (PC) als Material für die Folie verwendet. Bei alternativen bevorzugten Ausführungsformen wird Cyclo-Olefin-Copolymer (COC), Styrol-Acylnitril (SAN) oder Polystyrol (PS) als Material für die Folie verwendet. Bei weiteren alternativen bevorzugten Ausführungsformen wird ein bioabbaubares Polymer, wie z. B. Polymilchsäure als Material für die Folie verwendet.

Die Kapillaren und die Kanäle im herzustellenden Formkörper sind bevorzugt in gleicher Weise wie bei dem nachzubildenden Gewebe angeordnet. Daher werden die Ausformungen der Form entsprechend dem nachzubildenden Gewebe erzeugt, was beispielsweise durch lithographische Methoden oder durch spanende Fertigung der Form erfolgen kann.

Der Druck wird bevorzugt mithilfe eines Gases auf die Folie übertragen.

Bei einfachen Ausführungsformen des erfindungsgemäßen Verfahrens werden beim Falten die grabenartigen Vertiefungen der einen Schicht durch ebene Abschnitte der darüber befindlichen Schicht entlang ihrer Erstreckungsrichtung geschlossen. Bei bevorzugten Ausführungsformen treffen jedoch zwei der grabenartigen Vertiefungen aus beiden Schichten aufeinander. Dabei wird durch das Falten jeweils eine der grabenartigen Vertiefungen einer der Schichten deckungsglich über einer der grabenartigen Vertiefungen der darüber befindlichen Schicht angeordnet, sodass die dazwischen gebildete Kapillare symmetrisch zwischen den beiden Schichten ausgebildet wird. Hierbei besitzen die grabenartigen Vertiefungen bevorzugt jeweils die Form eines halben Hohlzylinders, sodass die gebildete Kapillare angenähert die Form eines Hohlzylinders besitzt.

Die Mulden können auf unterschiedliche Weise geöffnet werden. Bei einer ersten bevorzugten Ausführungsform werden die Mulden an deren Böden dadurch geöffnet, dass sie soweit in die Folie eingeformt werden, dass die Folie an den Böden der Mulden aufreißt. Durch das Formen wird die Folie im Bereich der Böden immer dünner, bis sie aufreißt. Bei einer zweiten bevorzugten Ausführungsform werden die Mulden an deren Böden dadurch geöffnet, dass die Böden der Mulden in einem Ätzbad aufgelöst werden. Beispielsweise kann die ausgeformte Folie soweit in das Ätzbad getaucht werden, dass sich nur die Böden der Mulden im Ätzbad befinden und dadurch aufgelöst werden.

Bei besonders bevorzugten Ausführungsformen werden vor dem Falten der ausgeformten Folie biologische Zellen zumindest auf einer der beiden Seiten der ausgeformten Folie angeordnet. Somit befinden sich die biologischen Zellen nach dem Falten bereits in Hohlräumen zwischen den Schichten des Stapels, beispielsweise in den Kapillaren. Dadurch können die biologischen Zellen aufwandsarm in diese Hohlräume eingebracht werden. Der vergleichsweise einfach durchzuführende Faltvorgang behindert die Ansiedlung der biologischen Zellen zumeist nicht. Bevorzugt werden die biologischen Zellen vor dem Falten der ausgeformten Folie auf beiden Seiten der ausgeformten Folie angeordnet. Somit können die biologischen Zellen in die Kapillaren und auch in andere Hohlräume zwischen den Schichten des Stapels eingebracht werden; bevorzugt durch ein Einbringen von Zellsuspension über Kapillarkräfte. Bevorzugt werden unterschiedliche Arten von biologischen Zellen auf den beiden Seiten der ausgeformten Folie aufgebracht, um komplexe biologische Gewebe nachzubilden. Die biologischen Zellen können beispielsweise durch Hepatozyten, Endothelzellen, Kupffer-Zellen oder Stellatzellen gebildet sein.

Ergänzend werden nach dem Falten bevorzugt weitere biologische Zellen in den Stapel, d. h. in den Formkörper eingebracht. Beispielsweise können die biologischen Zellen in die Kanäle und/oder in die Kapillaren gepumpt oder eingespült werden.

Bei weiteren besonders bevorzugten Ausführungsformen des erfindungsgemäßen Verfahrens wird vor dem Ausformen der Folie mindestens eine Schicht aus einem zellattraktiven Stoff oder aus einem zellabweisenden Stoff auf die Ausformungen der Form aufgebracht. Der zellattraktive Stoff bzw. der zellabweisende Stoff wird dadurch beim Ausformen der Folie auf die Folie aufgebracht, sodass Bereiche auf der ausgeformten Folie definiert sind, welche die biologischen Zellen entweder anziehen oder abweisen. Es hat sich überraschend gezeigt, dass sich der zellattraktive Stoff bzw. der zellabweisende Stoff auch über die Form im erwärmten Zustand der Folie beim Ausformen der Folie auf die Folie aufbringen lässt, was den besonderen Vorteil aufweist, dass der zellattraktive Stoff bzw. der zellabweisende Stoff entsprechend der Struktur der Ausformungen auf die Folie aufgebracht wird. Hierdurch kann der zellattraktive Stoff bzw. der zellabweisende Stoff aufwandsarm und mit einer hohen Genauigkeit auf die Folie aufgebracht werden. Da die Form die Ausformungen für die grabenartigen Vertiefungen und Mulden aufweist, wird der zellattraktive Stoff bzw. der zellabweisende Stoff bevorzugt so aufgebracht, dass - je nachdem, ob es sich um eine Positivform oder Negativform handelt - entweder nur die Ausformungen oder nur die Bereiche der Form außerhalb der Ausformungen mit dem zellattraktiven Stoff bzw. dem zellabweisenden Stoff versehen werden. Somit kann beispielsweise erreicht werden, dass der zellattraktive Stoff nur in die grabenartigen Vertiefungen, d. h. in die Kapillaren gelangt, sodass sich nur dort die biologischen Zellen ansiedeln. Die Form bildet somit einen Stempel aus, wofür die Form aus einem flexiblen oder steifen Material, wie beispielsweise PDMS oder Silizium besteht.

Alternativ oder ergänzend wird bevorzugt nach dem Ausformen der Folie eine Schicht aus einem zellattraktiven Stoff oder aus einem zellabweisenden Stoff auf ausgeformten Folie aufgebracht; bevorzugt entweder nur in den grabenartigen Vertiefungen und/oder in den Mulden oder nur in den Bereichen außerhalb der grabenartigen Vertiefungen und/oder der Mulden.

Der zellattraktive Stoff ist bevorzugt durch ein Hydrogel, beispielsweise durch Kollagenhydrogel gebildet. Der zellabweisende Stoff ist bevorzugt durch ein Hydrogel, beispielsweise durch Poly-NIPAAm-Hydrogel gebildet.

Die Folie weist beim Bereitstellen eine Dicke von bevorzugt zwischen 1 µm und 1 mm, weiter bevorzugt zwischen 10 µm und 200 µm auf. Bei besonders bevorzugten Ausführungsformen des erfindungsgemäßen Verfahrens beträgt die Dicke der Folie beim Bereitstellen zwischen 20 µm und 80 µm.

Die Folie weist beim Bereitstellen eine Breite und eine Länge auf, die bevorzugt jeweils zwischen 1 cm und 50 cm, besonders bevorzugt zwischen 5 cm und 20 cm betragen.

Die Folie weist bevorzugt Poren auf, durch welche Stoffe, insbesondere Flüssigkeiten von oder zu den biologischen Zellen gelangen können. Die Poren weisen beim Bereitstellen einen Durchmesser von bevorzugt zwischen 10 nm und 10 µm auf. Bei besonders bevorzugten Ausführungsformen weisen die Poren in der Folie beim Bereitstellen einen Durchmesser zwischen 100 nm und 5 µm, weiter bevorzugt zwischen 500 nm und 2 µm auf.

Die Dichte der Poren in der Folie, d. h. die Anzahl der Poren je Fläche der Folie, beträgt beim Bereitstellen bevorzugt mindestens 10⁵ Poren je cm², besonders bevorzugt mindestens 10⁶ Poren je cm². Die Dichte der Poren in der Folie beträgt beim Bereitstellen bevorzugt höchstens 10⁷ Poren je cm².

In der Folie werden bevorzugt Elektroden und/oder Transistorstrukturen angeordnet, um die Elektroaktivität der biologischen Zellen im späteren Formkörper messen zu können.

Bei bevorzugten Ausführungsformen des erfindungsgemäßen Verfahrens wird die Folie beim Pressen mittelbar über eine Opferfolie mit dem Druck, insbesondere mit dem den Druck aufweisenden Gas beaufschlagt.

Die ausgeformte Folie wird vor dem Falten bevorzugt beschnitten, um ungewünschte Ränder zu entfernen.

Die Opferfolie weist beim Bereitstellen eine Dicke von bevorzugt zwischen 1 µm und 500 µm, besonders bevorzugt zwischen 10 µm und 100 µm auf. Bei besonders bevorzugten Ausführungsformen des erfindungsgemäßen Verfahrens weist die Opferfolie beim Bereitstellen eine Dicke zwischen 20 µm und 80 µm auf.

Die Opferfolie besteht bevorzugt aus einem Kunststoff, beispielsweise einem Fluorkarbon oder Silikon. Besonders bevorzugt besteht die Opferfolie aus Perfluorethylenpropylen (FEP) .

Die grabenartigen Vertiefungen weisen eine Breite von bevorzugt zwischen 1 µm und 1 mm; weiter bevorzugt zwischen 10 µm und 300 µm und besonders bevorzugt zwischen 50 µm und 150 µm auf.

Die Mulden weisen einen Durchmesser von bevorzugt zwischen 10 µm und 5 mm; besonders bevorzugt zwischen 100 µm und 1 mm auf.

Die grabenartigen Vertiefungen und die Mulden bzw. die daraus entstehenden Kapillaren und Kanäle weisen zur Nachbildung des biologischen Gewebes bevorzugt solche Anordnungen auf, wie sie auch im dem nachzubildenden biologischen Gewebe gegeben sind. Im Falle einer nachzubildenden Leberstruktur erstrecken sich jeweils mehrere der grabenartigen Ausformungen bevorzugt sternförmig von jeweils einer der geöffneten Mulden in der Erstreckungsebene der Folie. Entsprechend erstrecken sich jeweils mehrere der Kapillaren bevorzugt sternförmig von jeweils einem der Kanäle in der Erstreckungsebene der jeweiligen Schicht des Stapels. Auch sind die grabenartigen Ausformungen bevorzugt in Form eines Dreieckgitters in der Erstreckungsebene der Folie angeordnet. Entsprechend sind die Kapillaren bevorzugt in Form eines Dreieckgitters in der Erstreckungsebene der jeweiligen Schicht des Stapels angeordnet. Das Dreiecksgitter bildet bevorzugt ein hexagonales Gitter. Bevorzugt bildet jeweils eine der Mulden einen Kreuzungspunkt der grabenartigen Ausformungen. Entsprechend liegt jeweils einer der Kanäle bevorzugt im Kreuzungspunkt der Kapillaren.

Der erfindungsgemäße Formkörper ist mit dem erfindungsgemäßen Verfahren herstellbar.

Der erfindungsgemäße Formkörper dient zur Nachbildung einer Struktur eines biologischen Gewebes. Bei dem Formkörper handelt es sich bevorzugt um die Nachbildung einer Struktur einer Leber bzw. Leberstruktur, wie z. B. Lebersinusoide und Leberläppchen, einer Blut-Hirn-Schranke oder eines Muskels, wobei grundsätzlich auch viele andere Arten von biologischem Gewebe nachgebildet sein können.

Der erfindungsgemäße Formkörper umfasst eine Folie, die in Form eines Stapels aus mindestens zwei Schichten der Folie ausgebildet ist. Es handelt sich somit um übereinander angeordnete Folienabschnitte. Die Folie weist grabenartige Vertiefungen auf. Die grabenartigen Vertiefungen erstrecken sich in der Erstreckungsrichtung der Folie, d. h. senkrecht zur Stapelrichtung. Die grabenartigen Vertiefungen sind entlang ihrer Erstreckungssichtung durch die benachbarte der Schichten des Stapels geschlossen, sodass Kapillaren ausgebildet sind. Somit bildet die benachbarte Schicht, d. h. die im Stapel unmittelbar darüber oder darunter befindliche Schicht, einen Deckel für die jeweilige grabenartige Vertiefung, sodass der jeweilige Graben nach oben bzw. unten geschlossen ist und die Kapillare ausbildet. Die Kapillaren sind an ihren axialen Enden bevorzugt geöffnet. Die Folie weist zudem Mulden auf, die an ihren Böden geöffnet sind, sodass Stoffe durch die Mulden hindurchdringen können. Jeweils mehrere der Mulden sind innerhalb des Stapels übereinander angeordnet, sodass diese einen senkrecht zur Erstreckungsebene der Folie angeordneten Kanal ausbilden. Der eine oder die mehreren Kanäle erstrecken sich somit senkrecht zu den Kapillaren. Der eine bzw. die mehreren Kanäle grenzen jeweils an zumindest eine der Kapillaren an, sodass Stoffe von dem jeweiligen Kanal in die jeweils angrenzende Kapillare oder zurück strömen können. Die Schichten des Stapels sind an einer Kante des Stapels jeweils paarweise über ein in der Folie ausgebildetes Filmscharnier verbunden, welches durch Kerben in der Folie gebildet ist. Somit ist der Stapel bevorzugt durch eine einzige Folie gebildet. Diese einzige Folie wurde durch Faltung zu dem Stapel gefaltet, wobei die Schichten des Stapels durch einzelne Abschnitte der einzigen Folie gebildet sind.

Der erfindungsgemäße Formkörper weist bevorzugt auch solche Merkmale auf, die im Zusammenhang mit dem erfindungsgemäßen Verfahren und dessen bevorzugten Ausführungsformen angegeben sind.

Im erfindungsgemäßen Formkörper sind bevorzugt biologische Zellen angeordnet. Diesen Zellen ist über die Kapillaren und über die Kanäle ein Stoffaustausch ermöglicht, sodass die Zellen fortbestehen können und ihre spezifischen Funktionen erfüllen können. Die Folie ist bevorzugt porös, sodass den biologischen Zellen über die Poren ein weiterer Stoffaustausch ermöglicht ist.

Bevorzugt sind zwischen den Schichten des Stapels und/oder auf der Oberseite und/oder auf Unterseite des Stapels die biologischen Zellen einer ersten Art und in den Kapillaren die biologischen Zellen einer zweiten Art angesiedelt. Hierdurch können komplexe biologische Gewebe, wie z. B. Leberläppchen nachgebildet werden. In diesem Fall sind die biologischen Zellen der ersten Art bevorzugt durch Hepatozyten gebildet, während die biologischen Zellen der zweiten Art bevorzugt durch Endothelzellen gebildet sind.

Bevorzugt befindet sich ausschließlich in den Kapillaren und/oder in den Kanälen eine zellattraktive oder eine zellabweisende Schicht auf der Folie. Ergänzend oder alternativ befindet sich bevorzugt ausschließlich in Bereichen außerhalb der Kapillaren und/oder der Kanäle eine zellattraktive oder eine zellabweisende Schicht auf der Folie.

Weitere Vorteile, Einzelheiten und Weiterbildungen der Erfindung ergeben sich aus der nachfolgenden Beschreibung bevorzugter Ausführungsformen der Erfindung, unter Bezugnahme auf die Zeichnung. Es zeigen:
- Fig. 1:: einen Vorgang zum Pressen einer Folie in eine Form gemäß einer bevorzugten Ausführungsform des erfindungsgemäßen Verfahrens;
- Fig. 2:: die in Fig. 1 gezeigte Folie in einer abgewandelten Ausführungsform nach dem Ausformen;
- Fig. 3:: die in Fig. 2 gezeigte Folie vor einem Faltvorgang gemäß einer ersten bevorzugten Ausführung;
- Fig. 4:: die in Fig. 3 gezeigte Folie im gefalteten Zustand;
- Fig. 5:: die in Fig. 2 gezeigte Folie vor einem Faltvorgang gemäß einer zweiten bevorzugten Ausführung;
- Fig. 6:: die in Fig. 5 gezeigte Folie im gefalteten Zustand;
- Fig. 7:: die in Fig. 4 gezeigte gefaltete Folie mit eingebrachten biologischen Zellen;
- Fig. 8:: die in Fig. 1 gezeigte Form gemäß einer weiteren bevorzugten Ausführungsform;
- Fig. 9:: ein Aufbringen einer zellattraktiven Schicht auf die in Fig. 8 gezeigte Form;
- Fig. 10:: eine gemäß dem erfindungsgemäßen Verfahren nachzubildende Struktur einer Leber;
- Fig. 11:: einen durch das erfindungsgemäße Verfahren nachgebildeten Sinusoid eines Leberläppchens;
- Fig. 12:: die in Fig. 11 gezeigte Nachbildung in siebenfacher Ausführung;
- Fig. 13:: einen für die in Fig. 12 gezeigte Nachbildung verwendeter Stempel;
- Fig. 14:: einen weiteren durch das erfindungsgemäße Verfahren nachgebildeten Sinusoid eines Leberläppchens;
- Fig. 15:: die in Fig. 11 gezeigte Nachbildung in siebenfacher Ausführung; und
- Fig. 16:: einen für die in Fig. 15 gezeigte Nachbildung verwendeter Stempel.

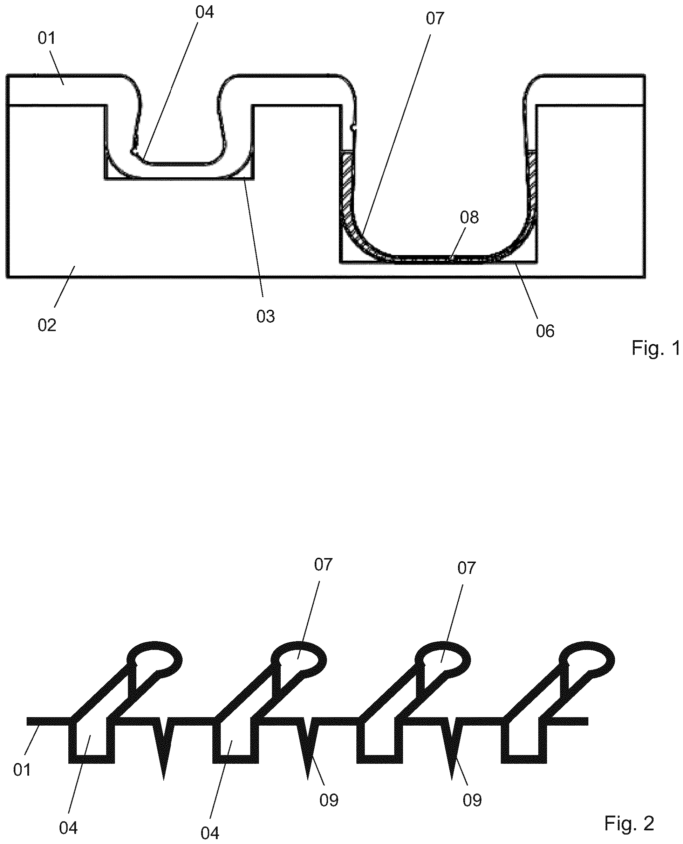

Fig. 1 zeigt einen Schritt einer bevorzugten Ausführungsform des erfindungsgemäßen Verfahrens. Bei diesem Schritt wird eine thermoplastische Folie 01 mithilfe eines Formgases (nicht dargestellt) in eine als Negativform ausgebildete Form 02 gepresst. Die Form 02 weist eine Ausformung 03 für eine grabenartige Vertiefung auf, sodass in der Folie 01 eine grabenartige Vertiefung 04 entsteht. Weiterhin weist die Form eine tiefere Ausformung 06 für eine Mulde auf, sodass in der Folie 01 eine Mulde 07 entsteht. Die Folie 01 wird soweit in die Ausnehmung 06 gepresst, bis die Folie 01 im Bereich eines Bodens 08 der Mulde 07 aufreißt. Zudem weist die Form Ausformungen (nicht dargestellt) für Kerben auf, sodass in der Folie 01 mehrere Kerben 09 (gezeigt in Fig. 2) entstehen.

Fig. 2 zeigt die in Fig. 1 gezeigte Folie 01 in einer abgewandelten Ausführungsform nach dem Ausformen in einer perspektivischen Teilansicht. Die Abwandlung besteht in der Anordnung der grabenartigen Vertiefungen 04, der Mulden 07 und der Kerben 09.

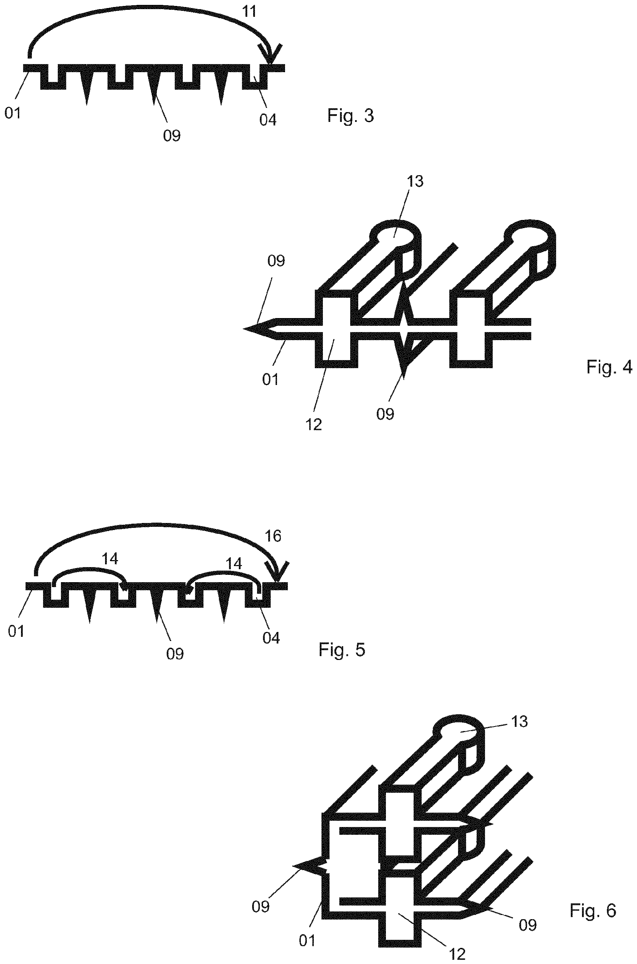

Fig. 3 zeigt die in Fig. 2 gezeigte Folie 01 vor einem Faltvorgang gemäß einer ersten bevorzugten Ausführung. Der auszuführende Faltvorgang ist durch einen Pfeil 11 symbolisiert. Das Falten erfolgt an der mittleren Kerbe 09, welche dabei als Filmscharnier fungiert.

Fig. 4 zeigt die in Fig. 3 gezeigte Folie 01 im gefalteten Zustand. Die eine Hälfte der Folie 01 wurde um 180° um die mittlere Kerbe 09 geschwenkt, sodass diese deckungsgleich auf die andere Hälfte der Folie 01 traf. Hierdurch wurden die grabenartigen Vertiefungen 04 längsseitig zusammengeführt, sodass diese jeweils paarweise eine Kapillare 12 ausbilden. Gleichzeitig wurden die geöffneten Mulden 09 übereinander gebracht, sodass sie paarweise jeweils einen Kanal 13 ausbilden, welcher sich senkrecht zu den Kapillaren 12 und zur Folie 01 erstreckt. Zwei der Kerben 09 bleiben bei dieser Ausführungsform ungenutzt. Die gefaltete Folie 01 bildet den erfindungsgemäß herzustellenden Formkörper.

Fig. 5 zeigt die in Fig. 2 gezeigte Folie 01 vor einem Faltvorgang gemäß einer zweiten bevorzugten Ausführung. Der auszuführende Faltvorgang umfasst einen ersten Schritt, welcher durch zwei Pfeile 14 symbolisiert ist, und einen zweiten Schritt, welcher durch einen Pfeil 16 symbolisiert ist. Das Falten erfolgt im ersten Schritt an den beiden äußeren Kerben 09, welche jeweils als Filmscharnier fungieren, und im zweiten Schritt an der mittleren Kerbe 09, welche dann ebenfalls als Filmscharnier fungiert.

Fig. 6 zeigt die in Fig. 5 gezeigte Folie 01 im gefalteten Zustand. Die beiden äußeren Viertel der Folie 01 wurden im ersten Schritt 14 (gezeigt in Fig. 5) um 180° um die beiden äußeren Kerben 09 um 180° nach innen geschwenkt, sodass diese deckungsgleich auf die beiden inneren Viertel der Folie 01 trafen. Hierdurch wurden die grabenartigen Vertiefungen 04 paarweise längsseitig zusammengeführt, sodass diese jeweils eine der Kapillaren 12 ausbilden. Gleichzeitig wurden die geöffneten Mulden 09 paarig übereinander gebracht, sodass sie einen Teil des Kanales 13 ausbilden, welcher sich senkrecht zu den Kapillaren 12 erstreckt. Im zweiten Schritt 16 (gezeigt in Fig. 5) wurden die beiden bereits gefalteten Hälften der Folie 01 um 180° um die innere Kerben 09 geschwenkt, sodass diese deckungsgleich aufeinander trafen. Hierdurch wurden die bereits ausgebildeten Teile des Kanales 13 übereinander gebracht, sodass der Kanal 13 vollständig ausgebildet wurde. Keine der Kerben 09 blieb bei dieser Ausführungsform ungenutzt. Die gefaltete Folie 01 bildet den erfindungsgemäß herzustellenden Formkörper.

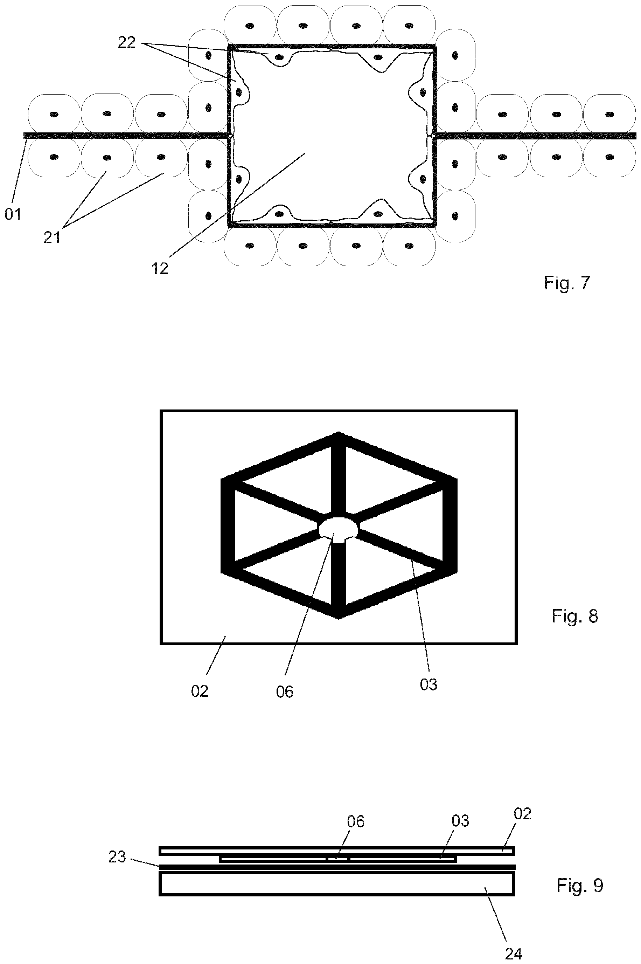

Fig. 7 zeigt die in Fig. 4 gezeigte gefaltete Folie 01, d. h. den erfindungsgemäß herzustellenden Formkörper in einer Detailansicht, bei welcher insbesondere eine der Kapillaren 12 im Querschnitt gezeigt ist. Diese Ausführungsform des erfindungsgemäßen Formkörpers dient der Nachbildung von Gewebe einer Leber, wofür biologische Zellen, nämlich Hepatozyten 21 und Endothelzellen 22 in den Formkörper eingebracht wurden. Die Endothelzellen 22 wurden in die Kapillare 12 eingebracht, während die Hepatozyten 21 in den Bereichen außerhalb der Kapillare 12 angesiedelt wurden. Das Ansiedeln der biologischen Zellen 21, 22 wird bevorzugt durch einen zellattraktiven Stoff 23 (gezeigt in Fig. 9) gesteuert.

Fig. 8 zeigt die in Fig. 1 gezeigte Form 02 in einer abgewandelten bevorzugten Ausführungsform, welche als Positivform insbesondere für die Herstellung des Formkörpers zur Nachbildung von Gewebe einer Leber geeignet ist. Hierfür weist die Form 02 die hexagonal angeordneten, erhabenen Ausformungen 03 für die grabenartigen Vertiefungen auf, in deren Zentrum die erhabene Ausformung 06 für die Mulde angeordnet ist. Die als Positivform ausgebildete Form 02 stellt somit einen Stempel dar.

Fig. 9 zeigt ein Aufbringen des zellattraktiven Stoffes 23 auf die in Fig. 8 gezeigte Form 02. Hierfür wird der zellattraktive Stoff 23 zunächst auf einer Unterlage 24 aus z. B. PDMS oder Glas angeordnet. Anschließend wird die Form 02 mit den erhabenen Ausformungen 03, 06 mit leichtem Druck auf den zellattraktiven Stoff 23 gepresst, wodurch dieser auf den erhabenen Ausformungen 03, 06 zum Anhaften kommt. Beim anschließenden Pressen der Folie 01 in die Form 02 (gezeigt in Fig. 1), was bei der hier gezeigten als Positivform ausgebildeten Form 02 durch ein Pressen der Form 02 auf die Folie 01 erfolgen kann, wird wiederum der zellattraktive Stoff 23 in die grabenartigen Vertiefungen 04 und in die Mulden 07 der Folie 01 (gezeigt in Fig. 1) gebracht. Die Bereiche außerhalb der grabenartigen Vertiefungen 04 und der Mulden 07 werden dabei nicht mit dem zellattraktiven Stoff 23 versehen.

Statt der Form 02 kann alternativ auch unmittelbar die geformte Folie 01 auf den zellattraktiven Stoff 23 auf der Unterlage 24 gepresst werden. Statt des zellattraktiven Stoffes 23 kann auch ein zellabweisender Stoff in der beschriebenen Weise auf die Form 02 bzw. auf die ausgeformte Folie 01 aufgebracht werden.

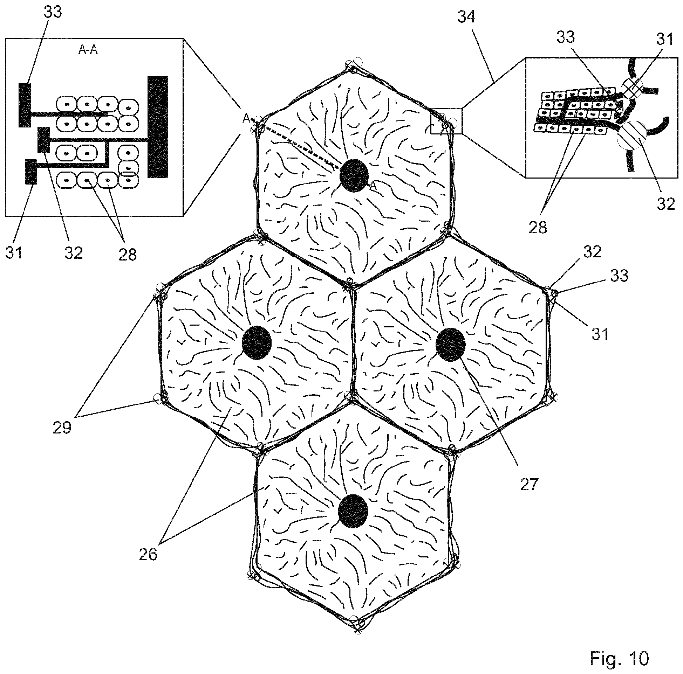

Fig. 10 zeigt eine gemäß dem erfindungsgemäßen Verfahren nachzubildende Struktur einer Leber. Die Struktur umfasst vier hexagonal ausgebildete Leberläppchen 26, in deren Zentren sich jeweils eine Vena centralis 27 befindet. In einem Schnitt A-A sind insbesondere Hepatozyten 28 dargestellt. An den äußeren Ecken der Leberläppchen 26 befindet sich jeweils ein Glisson-Trias 29 mit einer Arteria interlobularis 31, einer Vena interlobularis 32 und einer Doctuli interlobularis 33, die insbesondere in einer Detaildarstellung 34 eines der Glisson-Trias 29 gezeigt sind.

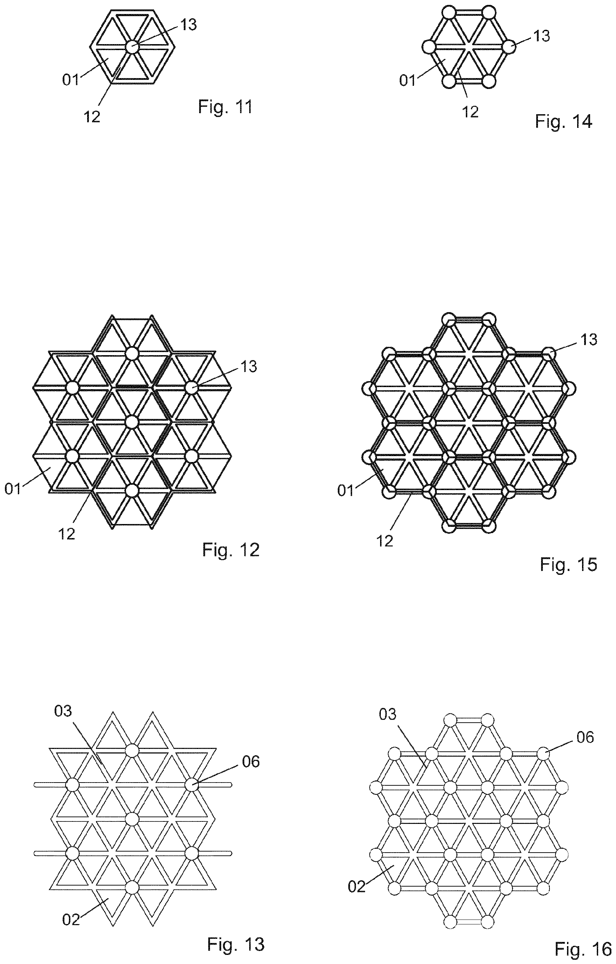

Fig. 11 zeigt einen durch das erfindungsgemäße Verfahren nachgebildeten Sinusoid eines der Leberläppchen 26 (gezeigt in Fig. 10) mit dem Kanal 13 zur Nachbildung der Vena centralis 27 (gezeigt in Fig. 10).

Fig. 12 zeigt die in Fig. 11 dargestellte Nachbildung in siebenfacher Ausführung in hexagonaler Anordnung, sodass ein größerer Teil des Gewebes einer Leber nachgebildet ist.

Fig. 13 zeigt die für die in Fig. 12 dargestellte Nachbildung verwendete Form 02, welche als Stempel ausgebildet ist. Die Form 02 weist die Ausformungen 03 für die grabenartigen Vertiefungen und die Ausformungen 06 für die Mulden auf.

Fig. 14 zeigt einen weiteren durch das erfindungsgemäße Verfahren nachgebildeten Sinusoid eines der Leberläppchen 26 (gezeigt in Fig. 10) mit den mehreren Kanälen 13 zur Nachbildung der Glisson-Trias 29 (gezeigt in Fig. 10).

Fig. 15 zeigt die in Fig. 14 dargestellte Nachbildung in siebenfacher Ausführung in hexagonaler Anordnung, sodass ein größerer Teil des Gewebes einer Leber nachgebildet ist.

Fig. 16 zeigt die für die in Fig. 15 dargestellte Nachbildung verwendete Form 02, welche als Stempel ausgebildet ist. Die Form 02 weist die Ausformungen 03 für die grabenartigen Vertiefungen und die Ausformungen 06 für die Mulden auf.

### Bezugszeichenliste

- 01: Folie
- 02: Form
- 03: Ausformung für grabenartige Vertiefung
- 04: grabenartige Vertiefung
- 05: -
- 06: Ausformung für Mulde
- 07: Mulde
- 08: Boden
- 09: Kerbe
- 10: -
- 11: Pfeil
- 12: Kapillare
- 13: Kanal
- 14: Pfeile
- 15: -
- 16: Pfeil

- 21: Hepatozyten
- 22: Endothelzellen
- 23: zellattraktiver Stoff
- 24: Unterlage
- 25: -
- 26: Leberläppchen
- 27: Vena centralis
- 28: Hepatozyten
- 29: Glisson-Trias
- 30: -
- 31: Arteria interlobularis
- 32: Vena interlobularis
- 33: Doctuli interlobularis
- 34: Detail

## Patentansprüche

1. Verfahren zur Herstellung eines Formkörpers zur Nachbildung einer Struktur eines biologischen Gewebes, folgende Schritte umfassend:
- Bereitstellen einer plastisch verformbaren Folie (01);
- Beaufschlagen der Folie (01) mit einem Druck, um diese in eine Form (02) zu pressen, wobei die Form (02) Ausformungen (03, 06) für grabenartige Vertiefungen (04), für Mulden (07) und für Kerben (09) aufweist, wobei die grabenartigen Vertiefungen (04) in der Erstreckungsebene der Folie (01) ausgeformt werden, wobei die Mulden (07) jeweils zumindest an eine der grabenartigen Vertiefungen (04) angrenzen und jeweils einen Boden (08) aufweisen, an welchem sie geöffnet werden, sodass jeweils eine Öffnung entsteht, und wobei die Kerben (09) mindestens ein Filmscharnier in der Folie (01) ausbilden; und
- Falten der ausgeformten Folie (01) zu einem mindestens zwei Schichten der Folie (01) umfassenden Stapel, wobei das Filmscharnier die Faltkante zum Falten bildet, wobei die grabenartigen Vertiefungen (04) entlang ihrer Erstreckungsrichtung durch eine benachbarte der Schichten des Stapels geschlossen werden und jeweils eine Kapillare (12) ausbilden, und wobei mindestens zwei der geöffneten Mulden (07) übereinander angeordnet werden und einen senkrecht zur Erstreckungsebene der Folie (01) angeordneten Kanal (13) ausbilden.

2. Verfahren nach Anspruch 1, **dadurch gekennzeichnet, dass** die Folie (01) vor dem Pressen in die Form (02) auf eine Temperatur zwischen 140 °C und 180 °C erwärmt wird.

3. Verfahren nach Anspruch 1 oder 2, **dadurch gekennzeichnet, dass** jeweils eine der grabenartigen Vertiefungen (04) einer der Schichten deckungsgleich über einer der grabenartigen Vertiefungen (04) der darüber befindlichen Schicht angeordnet wird, sodass die dazwischen gebildete Kapillare (12) symmetrisch zwischen den beiden Schichten ausgebildet wird.

4. Verfahren nach einem der Ansprüche 1 bis 3, **dadurch gekennzeichnet, dass** die Mulden (07) an deren Böden (08) dadurch geöffnet werden, dass sie soweit in die Folie (01) eingeformt werden, dass die Folie (01) an den Böden (08) der Mulden (07) aufreißt.

5. Verfahren nach einem der Ansprüche 1 bis 3, **dadurch gekennzeichnet, dass** die Mulden (07) an deren Böden (08) dadurch geöffnet werden, dass die Böden (08) der Mulden (07) in einem Ätzbad aufgelöst werden.

6. Verfahren nach einem der Ansprüche 1 bis 5, **dadurch gekennzeichnet, dass** vor dem Falten der ausgeformten Folie (01) folgender Schritt durchgeführt wird:
- Ansiedeln von biologischen Zellen (21, 22) zumindest auf einer der beiden Seiten der ausgeformten Folie (01).

7. Verfahren nach einem der Ansprüche 1 bis 6, **dadurch gekennzeichnet, dass** die Folie (01) mittelbar über eine Opferfolie mit dem Druck beaufschlagt wird.

8. Verfahren nach einem der Ansprüche 1 bis 7, **dadurch gekennzeichnet, dass** die Folie (01) beim Bereitstellen Poren aufweist, welche einen Durchmesser zwischen 500 nm und 2 µm besitzen.

9. Formkörper zur Nachbildung einer Struktur eines biologischen Gewebes, umfassend eine Folie (01), die in Form eines Stapels aus mindestens zwei Schichten ausgebildet ist;
- wobei die Folie (01) grabenartige Vertiefungen (04) aufweist, die entlang ihrer Erstreckungsrichtung durch eine benachbarte der Schichten des Stapels geschlossen sind, sodass Kapillaren (12) ausgebildet sind;
- wobei die Folie (01) Mulden (07) aufweist, die an ihren Böden (08) geöffnet sind, wobei mehrere der Mulden (07) innerhalb des Stapels übereinander angeordnet sind, sodass durch die jeweils übereinander angeordneten geöffneten Mulden (07) ein senkrecht zur Erstreckungsebene der Folie (01) angeordneter Kanal (13) ausgebildet ist, welcher zumindest an eine der Kapillaren (12) angrenzt; und
- wobei die Schichten an einer Kante des Stapels jeweils paarweise über ein in der Folie (01) ausgebildetes Filmscharnier verbunden sind, welches durch Kerben (09) in der Folie (01) gebildet ist.

## Claims

1. Method for producing a molding for replicating a structure of a biological tissue, comprising the following steps:
- Providing of a plastically deformable film (01);
- Subjecting the film (01) to a pressure in order to press it into a mold (02), the mold (02) comprising formations (03, 06) for pit-like depressions (04), for recesses (07) and for notches (09), wherein the pit-like depressions (04) are formed in the plane of extension of the film (01), while the recesses (07) each border on at least one of the pit-like depressions (04) and each have a bottom (08), at which they are opened, so that each time an opening is produced, and the notches (09) form at least one film hinge in the film (01); and
- Folding of the formed film (01) to produce a stack comprising at least two layers of the film (01), the film hinge forming the folding edge for the folding, whereby the pit-like depressions (04) are closed along their direction of extension by a neighboring layer of the stack and form a capillary (12), and whereby at least two of the opened recesses (07) are arranged one on top of the other and form a canal (13) arranged perpendicular to the plane of extension of the film (01) .

2. Method according to Claim 1, **characterized in that** the film (01) is heated prior to the pressing into the mold (02) to a temperature between 140°C and 180°C.

3. Method according to Claim 1 or 2, **characterized in that** each time one of the pit-like depressions (04) of one of the layers is arranged congruently across one of the pit-like depressions (04) of the layer located above it, so that the capillary (12) formed between them is symmetrically formed between the two layers.

4. Method according to one of Claims 1 to 3, **characterized in that** the recesses (07) are opened up at their bottoms (08) in that they are molded far enough into the film (01) that the film (01) rips at the bottom (08) of the recesses (07).

5. Method according to Claims 1 to 3, **characterized in that** the recesses (07) are opened up at their bottoms (08) by dissolving the bottoms (08) of the recesses (07) in an etching bath.

6. Method according to one of Claims 1 to 5, **characterized in that** prior to the folding of the shaped film (01) the following step is carried out:
- Colonizing of biological cells (21, 22) at least on one of the two sides of the shaped film (01).

7. Method according to one of Claims 1 to 6, **characterized in that** the film (01) is subjected to the pressure indirectly through a sacrifice film.

8. Method according to one of Claims 1 to 7, **characterized in that** the provided film (01) has pores with a diameter between 500 nm and 2 µm.

9. Molding for replicating a structure of a biological tissue, comprising a film (01), which is configure in the form of a stack of at least two layers;
- Wherein the film (01) has pit-like depressions (04), which are closed along their direction of extension by a neighboring layer of the stack, so that capillaries (12) are formed;
- Wherein the film (01) has recesses (07), which are open at heir bottoms (08), while several of the recesses (07) are arranged one on top of another within the stack, so that a canal (13) arranged perpendicular to the plane of extension of the film (01) is formed by the open recesses (07) arranged one on top of another, which borders at least on one of the capillaries (12); and
- Wherein the layers are connected in pairs at one edge of the stack by a film hinge formed in the film (01), which is formed by notches (09) in the film (01).

## Revendications

1. Procédé de fabrication d'un corps moulé pour reproduire une structure d'un tissu biologique, comprenant les étapes suivantes :
- fournir un film (01) déformable plastiquement ;
- appliquer une pression sur le film (01) pour comprimer celui-ci en dans un moule (02), dans lequel le moule (02) présente des conformations (03, 06) pour des renfoncements (04) de type tranchée pour des creux (07) et pour des entailles (09), dans lequel les renfoncements (04) de type tranchée sont formés dans le plan d'étirement du film (01), dans lequel les creux (07) sont respectivement délimités au moins contre un des renfoncements (04) de type tranchée et présentent chacun un fond (08) sur lequel ils sont ouverts, de façon à ce que respectivement une ouverture soit produite, et dans lequel les entailles (09) forment au moins une jointure de film dans le film (01) ; et
- de plier le film formé (01) en une pile comprenant au moins deux couches du film (01), dans lequel la jointure de film forme l'arête de pliage pour le pliage, dans lequel les renfoncements (04) de type tranchée sont fermés le long de leur sens d'étirement par une voisine des couches de la pile et forment respectivement un capillaire (12), et dans lequel au moins deux des creux (07) ouverts sont disposés l'un sur l'autre et forment un canal (13) disposé verticalement au plan d'étirement du film (01).

2. Procédé selon la revendication 1, **caractérisé en ce que** le film (01) est chauffé à une température entre 140°C et 180°C avant la compression dans le moule (02) .

3. Procédé selon la revendication 1 ou 2, **caractérisé en ce que** respectivement un des renfoncements (04) de type tranchée d'une des couches est disposé en coïncidant sur un des renfoncements (04) de type tranchée de la couche se trouvant dessus, de sorte que le capillaire (12) formé entre est formé symétriquement entre les deux couches.

4. Procédé selon l'une des revendications 1 à 3, **caractérisé en ce que** les creux (07) sont ainsi ouverts sur leurs fonds (08) qu'ils sont formés si loin dans le film (01) que le film (01) se déchire sur les fonds (08) des creux (07).

5. Procédé selon l'une des revendications 1 à 3, **caractérisé en ce que** les creux (07) sont ainsi ouverts sur leurs fonds (08) que les fonds (08) des creux (07) sont dissous dans un bain caustique.

6. Procédé selon l'une des revendications 1 à 5, **caractérisé en ce qu'**avant le pliage du film (01) formé, l'étape suivante est exécutée :
- implantation de cellules biologiques (21, 22) au moins sur un des deux côtés du film (01) formé.

7. Procédé selon l'une des revendications 1 à 6, **caractérisé en ce que** le film (01) reçoit la pression indirectement par un film sacrificiel.

8. Procédé selon l'une des revendications 1 à 7, **caractérisé en ce que** le film (01) présente à la fourniture des pores qui ont un diamètre entre 500 nm et 2 µm.

9. Corps moulé pour reproduire une structure d'un tissu biologique, comprenant un film (01) qui est sous forme d'une pile d'au moins deux couches ;
- dans lequel le film (01) présente des renfoncements (04) de type tranchée qui sont fermés le long de leur sens d'étirement par une voisine des couches de la pile, de façon à former des capillaires (12) ;
- dans lequel le film (01) présente des creux (07) qui sont ouverts sur leurs fonds (08), dans lequel plusieurs des creux (07) sont disposés l'un sur l'autre à l'intérieur de la pile, de sorte que par les creux (07) ouverts disposés l'un sur l'autre respectivement, un canal (13) disposé verticalement au plan d'étirement du film (01) est formé, lequel est délimité au moins contre un des capillaires (12) ; et
- dans lequel les couches sont reliées sur une arête de la pile respectivement par paires par une jointure de film formée dans le film (01) qui est formée par des entailles (09) dans le film (01) .
